# EUROPEAN PATENT SPECIFICATION

(11) **EP 0 976 072 B1**
(45) Date of publication and mention of the grant of the patent: **25.05.2005**
(21) Application number: 99902733.7
(22) Date of filing: 11.02.1999
(51) Int. Cl.: G06F 17/30

(54) **INDEXING AND WEB-TECHNOLOGIES CONTROL INTERACTING WITH DATABASE**
INDEXIERUNGS UND NETZTECHNOLOGIEKONTROLLE WECHSELSEITIG WIRKEND MIT DATENBANK
INTERACTION D'UNE BASE DE DONNEES AVEC LA GESTION DE L'INDEXATION ET DES TECHNIQUES LIEES A L'INTERNET

(30) Priority: 18.02.1998 US 25376
(43) Date of publication of application: 02.02.2000
(73) Proprietor: Koninklijke Philips Electronics N.V., 5621 BA Eindhoven (NL); PHILIPS AB, 164 85 Stockholm (SE)
(72) Inventor: BAR-GADDA, Uzi, NL-5656 AA Eindhoven (NL); WONG, Stephen, NL-5656 AA Eindhoven (NL); YU, James, Y-P., NL-5656 AA Eindhoven (NL)
(74) Representative: Cohen, Julius Simon
(86) International application number: PCT/IB1999/000240
(87) International publication number: WO 1999/042933

(56) References cited:
- EP-A2- 0 849 689
- WO-A1-96/31826
- JP-A- 11 015 716

## Description

### FIELD OF THE INVENTION

The invention relates to a method for accessing, through a client-server architecture, multimedia data distributed across heterogeneous databases. The invention relates in particular to such a method within an electronic medical record (EMR) environment.

### BACKGROUND ART

The ongoing development in the medical fields of diagnostics and therapy has led to an ever increasing number of techniques, and to dramatic changes in the delivery of health care and in the payment for health care services. Medical documents have become inherently multimedia, ranging from structured written reports and free text to non-alphanumeric data such as medical images, video streams, physiologic monitoring signals, voice dictation and graphics. The result of all this is that the complexity of managed care and of patient information has expanded greatly. Paper- and film-based recording systems and isolated, individual departmental information managing systems seem to have reached the point that they are no longer adequate in meeting the needs of integrated care.

### OBJECT OF THE INVENTION

Hospitals and care provider organizations need a new information model to communicate, distribute, and manage correlated patient information items in an efficient and cost-effective manner, while maintaining or even improving the quality of care. Therefore, it is an object of the invention to provide a method of managing interaction with electronic record databases.

### SUMMARY OF THE INVENTION

To this end, the invention provides a method of interacting between multiple users through an electronic records database system. A first user sends to a second user an electronic notification having a pointer to a record. The notification is being delivered through a push technology. The second user uses the pointer to retrieve the record through a pull technology. The notification is being indexed in the database as related to the record. If the first user sends an electronic message in response to the notification, to e.g., a third user, the message is indexed in the database as related to the record. Preferably, the notification is customized according to a specification by the second user with regard to a modality of reception by the second user, the specification having been stored in the database. The modality may relate to, e.g., electronic receiving equipment of the second user, or to a temporal relevance of the notification.

The invention is based on the following insights. The World Wide Web (or the Web) is the vast collection of electronic information resources accessible on the Internet through a client-server architecture. A client-server computer architecture is an information processing system with a server computer that enables processing power, data files and program files to be shared among multiple client computers interconnected to the server through a network. The Web is interacted with typically using a pull technology. The term "pull technology" refers to the mode of interaction according to which the user clicks the links on the Web to request the server to send information. That is, the user has to request for each piece of information that he/she needs to access, typically after querying the Web. Browsing the Web and using bookmarks are examples of the pull model. On the other hand, "push-technology" is data distribution according to which selected data is delivered automatically to the user's computer. This type of delivery is also referred to as "webcasting". Delivery of data can be at prescribed intervals or can be based on some prescribed events. An example of push-technology is PointCast, which is an Internet service for periodically pushing selected news and stock quotes into a subscriber's machine. Marimba's Castanet provides a push delivery for updating applications as well as distributing publishing content.

The invention now combines push-technology and pull-technology and automatic indexing services in a single architecture. The architecture creates an efficient procedure to manage and access the large numbers of records distributed among multiple locations and available in a variety of formats on a web-based networked system. The messaging between users is automatically indexed to keep the database up to date. For relevant Web-related technologies, especially as applied to the medical environment, see for example, U.S. patent publication No US 6 557 102, US 5 930 804, US 6 076 166.

Document WO 96/31826 discloses a method of processing messages which include links to further information.

### BRIEF DESCRIPTION OF THE DRAWINGS

The invention is explained by way of example and with reference to the accompanying drawings, wherein:
Fig.1 is a block diagram of a system architecture in the invention;
Fig.2 is a workflow diagram illustrating the messaging procedure in the system.

Throughout the figures, same reference numerals indicate similar or corresponding features.

### PREFERRED EMBODIMENTS

An example of a field of application in the invention relates to medical records. The current trend in managing electronic medical records is towards distribution across networked information systems, especially as driven by the Integrated Delivery Network (IDN) in the healthcare industry. Tracking of the records becomes one of the largest overheads in the medical environment. Due to the huge amount of data including audio, video, graphics images and text reports, some or all involving mission critical criteria, current pushing technology is not appropriate. According to the invention, by using the Web-based technologies the medical specialist can subscribe to receipt of the required data. The indexing to those data is organized and pushed to those subscribers whenever it is available. The subscribers can set the criteria on the relevance of the data. The subscribers are notified by this system when the deadline to deliver the data has passed because of the unavailability of the data. The subscribers can be notified according to their preferences listed in a user personalization profile. More specifically, the user can personalize the way wherein he or she interacts with the system through push technology and pull technology. For example, the user can specify what information is to be received, how to get the information (pager, email, voice, etc.). This is illustrated below.

### Block diagram

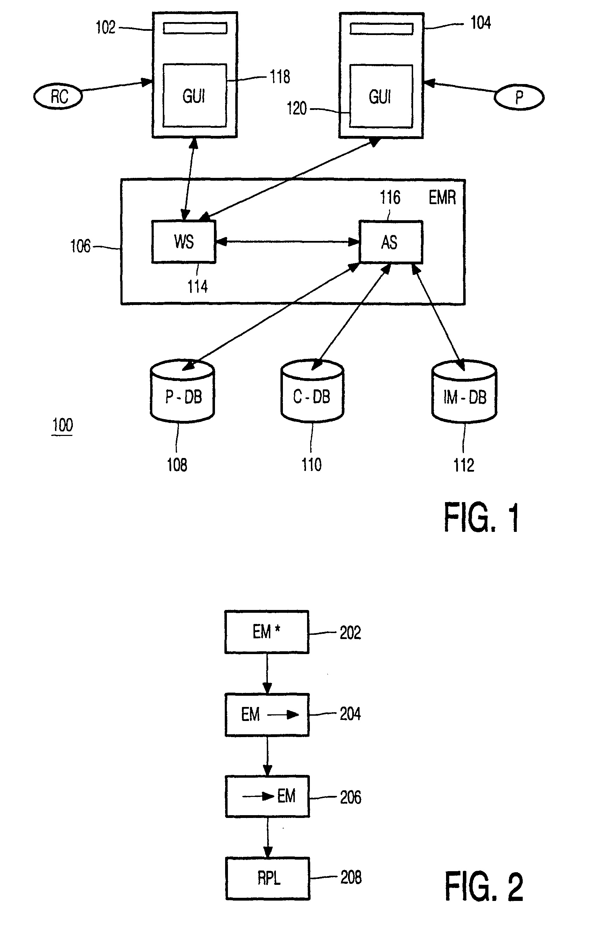

Fig.1 is a block diagram of a system 100 according to the invention. System 100 supports automatic notification and indexing services of patient records and consultation notes. In case of an event that needs attention from clinical personnel, system 100 notifies one or more specific users through push technology, for example, by means of selective transmission of emails that have pointers to relevant pages of medical records accessible on the Web. The specific user can then pull the medical records by clicking the pointers. To this end, system 100 comprises client devices, e.g., devices 102 and 104, server equipment 106 and databases 108, 110 and 112. Devices 102-104 communicate with databases 108-112 via server equipment 106. Server equipment 106 has a Web server (WS) 114 and an application server (AS) 116. Web server 114 is a computer running a server program whose sole purpose is to serve document data to a client computer, such as device 102 or 104, in response to receiving a request for that document. Application server 116 is a computer that provides access to application software. An action specified at client device 102 or 104 can run an application on application server 116. Devices 102-104 comprise Web browsers 118 and 120, respectively, each with an appropriate GUI. Devices 102-104 comprise, for example, a desktop PC, a palmtop PC, a PDA, a wireless device, a set-top box, or any other device with Web browser capability, a pager, or a telephone answering machine. Database 108 comprises a personalization database (P-DB) that contains the user preferences and access privilege information as explained below. Database 110 comprises one or more clinical databases (C-DB) that store patient records and images. Database 112 comprises an indexed mail database IM-DB that contains formatted email messages and indices (pointers, links) to large, relevant patient data and images referred to in the email messages and archived in clinical database 110.

Application server 116 automatically keeps track of the events in databases 108-112, e.g., update of medical records of specific patient. Server 116 notifies the involved clinicians with email messages (i.e. through push technology) that comprise embedded links or pointers to large patient data files in database 110 (i.e., retrievable through pull technology). Besides using emails for notification, application server 116 can also page or make a telephone call to the relevant users. The pager numbers and telephone numbers of the users are stored in personalization database 108. A simple memo in the form of an alphanumeric message can be sent to the receiver's pager along with source contact information, such as the phone number of pager number of another clinician. For voice messaging, application server 116 synthesizes a voice by translating textual messages into audio signals and dials the telephone numbers of the relevant receivers. Which way for notification is being used is, in this example, user-dependent and can be specified in advance by storing user-preferences in personalization database 108.

### Workflow procedure

Operation of system 100 is illustrated below with reference to Fig.2, discussing the procedural steps of electronic communication of personalized medical records for a clinical consultation.

In step 202 an email message is constructed. For example, a patient consults a physician and after the patient encounter, the physician sends one or more email messages to one or more specific clinicians, e.g., a specialist and a nurse. The email relates to multimedia data, including graphics, audio and video. Such an email message contains a first part and a second part. The first part has a clinical statement on the patient and contains one or more links to be clicked on to retrieve relevant patient data stored in database 110. The second part lists action items requested by the referring physician. For example, the clinical statement of the email message contains observation data resulting from the consult and, in addition, a request for an X-ray study on the patient and pointers to previous X-ray reports and images of the patient relevant to the case. An action item is for receipt by a referred radiologist to perform an X-ray study on the patient and requests to send a diagnosis immediately back to the referring physician for prognosis.

In step 204 the email message is delivered to the referred clinicians. There are a variety of ways to deliver the message, depending on, for example, the urgence of the case. First, the referring clinician can request system 100 to page the receiver once the message has been sent. Second, the referring clinician can specify the time to page the receiver before the occurrence of a certain event. For example, two hours before a scheduled surgery on the patient the radiologist would like to ensure the surgeon to read the diagnostic interpretation of the latest MRI study performed on that patient. Third, the referring physician simply sends the message without any specified period of notification, as in typical email use. On the other hand, a clinician can specify in personalization database 108 how long before a certain event he or she would like to be notified.

In step 206 the email message is received and relevant data is retrieved. The referred clinician receives the email message, studies the attached observations and clicks on the relevant links in order to retrieve patient data of interest for review, e.g., previous diagnostic reports of the patient's X-ray studies. The referred clinician then responds to the request of the referring physician, e.g., by interpreting the results of the latest X-ray study on the patient. The formal report can be updated in database 110 using existing procedures for hospital information systems. However, for fast response and additional comments outside the formal reporting structure, the referred clinician can append summaries or comments of the study to the initiator's email and forward them to the initiator and to other relevant parties. Email database 112 keeps an audit trail of such messages.

In step 208, a message folder reply (RPL) is created and forwarded. The referred clinician composes the reply message with links to new of other relevant patient records in system 100 and delivers the message to the referring physician or forwards the message to the next care provider for the patient in this particular encounter according to procedural step 204.

### GUI for Web Browser

The Table presents a view of a GUI for a Web browser for display on a Web-enabled device 102, such as desktop PC, a palmtop PC, e.g., the Velo-1 of Philips Electronics, or a digital TV screen.

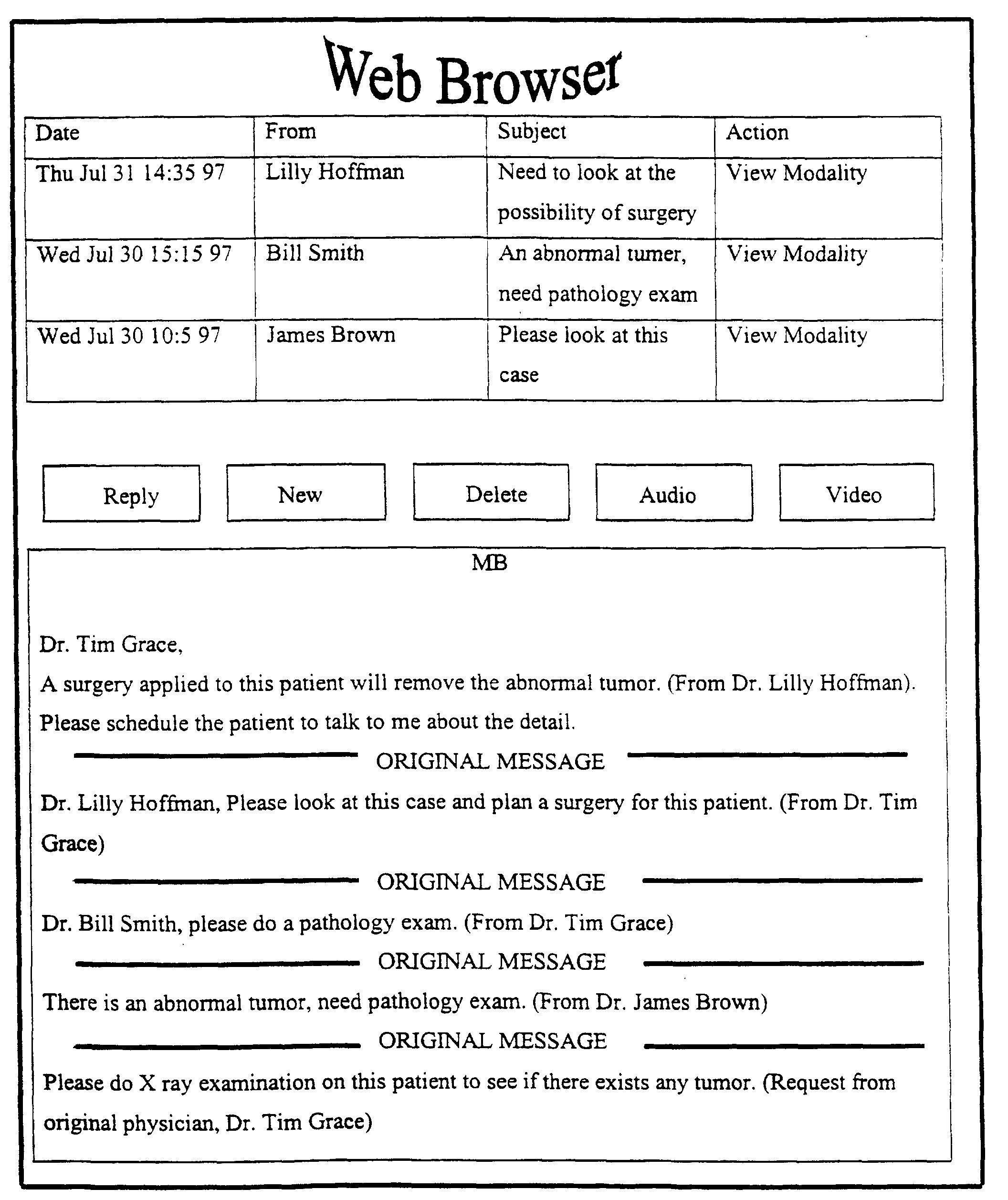

GUI has fields with labels "Date", "From", "Subject", and "Action", respectively. The Date Field indicates the date and time on which a specific email was sent. The From-Field indicates the name of the sender of the email. The Subject-Field contains the subject of the email. When the user clicks on the underlined subject, GUI shows the email message in a message box MB. The Action-Field contains the underlined pointer to pull relevant patient records from database 110, including diagnosis reports and images. When the user clicks on the pointer, the related reports and images are retrieved from database 110 and are presented to the user through a Web browser application or Java-based applets.

GUI has also buttons, labeled "Reply", "New", "Delete", "Audio" and "Video", respectively. When button is clicked, the original message in the image box is attached to a reply message that the user can enter and send to other users. When the New-button is clicked, the message in the message box is cleared, and the user can enter a new message and specify subject and receiver. The link to the current patient case, which the user is looking at, is attached as the "Action" message. When the Delete-button is clicked the currently selected (i.e., viewed) message is deleted and the next message on the list is retrieved and shown in the message box. When the user clicks on the Audio-button, a new message will be created by recording the dictation from the user, employing a suitable speech-to-text conversion program, or the audio message is stored and forwarded as an audio message (i.e., without using speech-to-text conversion). When the Video-button is clicked, the user is enabled to record his/her video and/or audio data to be sent to the receiver.

## Claims

1. A method of interacting between multiple users through an electronic records database system, wherein:
- a first user sends to a second user an electronic notification having a pointer to a record, **characterized by**
- the notification is being delivered through a push technology;
- the second user uses the pointer to retrieve the record through a pull technology; and
- the notification is being indexed in the database system as related to the record.

2. The method of claim 1, wherein:
- the first user sends an electronic message in response to the notification; and
- the message is indexed in the database system as related to the record.

3. The method of claim 1, wherein
- the interacting is personalized according to a specification by the second user with regard to a modality of reception by the second user; and
- the specification has been stored in the database system.

4. The method of claim 3, wherein the modality relates to a capability of electronic receiving equipment of the second user.

5. The method of claim 3, wherein the modality relates to a temporal relevance of the notification.

## Patentansprüche

1. Verfahren der Wechselwirkung zwischen mehreren Benutzern über ein elektronisches Aufzeichnungsdatenbanksystem, wobei
- ein erster Benutzer einem zweiten Benutzer eine elektronische Benachrichtigung mit einem Zeiger auf eine Aufzeichnung sendet, **dadurch gekennzeichnet, dass**
- die Benachrichtigung mittels einer Push-Technik zugestellt wird;
- der zweite Benutzer den Zeiger nutzt, um die Aufzeichnung durch eine Pull-Technik abzurufen; und
- die Benachrichtigung in dem Datenbanksystem so indexiert wird, dass sie mit der Aufzeichnung verknüpft ist.

2. Verfahren nach Anspruch 1, wobei
- der erste Benutzer eine elektronische Nachricht als Antwort auf die Benachrichtigung sendet; und
- die Nachricht in dem Datenbanksystem so indexiert wird, dass sie mit der Aufzeichnung verknüpft ist.

3. Verfahren nach Anspruch 1, wobei
- die Wechselwirkung gemäß einer Spezifikation durch den zweiten Benutzer im Hinblick auf eine Empfangsmodalität des zweiten Benutzers personalisiert wird; und
- die Spezifikation in dem Datenbanksystem gespeichert wurde.

4. Verfahren nach Anspruch 3, wobei sich die Modalität auf eine Fähigkeit der elektronischen Empfangsvorrichtung des zweiten Benutzers bezieht.

5. Verfahren nach Anspruch 3, wobei sich die Modalität auf eine zeitliche Relevanz der Benachrichtigung bezieht.

## Revendications

1. Procédé d'interaction entre des utilisateurs multiples à travers un système de bases de données d'enregistrements électroniques dans lequel :
- un premier utilisateur envoie à un deuxième utilisateur une notification électronique comportant un pointeur vers un enregistrement, **caractérisée en ce que**
- la notification est délivrée selon une technologie du pousser;
- le deuxième utilisateur utilise le pointeur pour récupérer l'enregistrement selon une technologie du tirer; et
- la notification est indexée dans le système de bases de données comme étant liée à l'enregistrement.

2. Procédé selon la revendication 1 dans lequel :
- le premier utilisateur envoie un message électronique en réponse à la notification; et
- le message est indexé dans le système de bases de données comme étant lié à l'enregistrement.

3. Procédé selon la revendication 1 dans lequel :
- l'interaction est personnalisée en fonction d'une spécification par le deuxième utilisateur à propos d'une modalité de réception par le deuxième utilisateur; et
- la spécification a été mémorisée dans le système de bases de données.

4. Procédé selon la revendication 3 dans lequel la modalité se rapporte à une fonctionnalité de l'équipement électronique de réception dont dispose le deuxième utilisateur.

5. Procédé selon la revendication 3 dans lequel la modalité se rapporte à une pertinence temporelle de la notification.
